(19) Europäisches Patentamt
European Patent Office
Office européen des brevets

(11) **EP 3 589 270 B1**

(12) **EUROPÄISCHE PATENTSCHRIFT**

(45) Veröffentlichungstag und Bekanntmachung des Hinweises auf die Patenterteilung:
**19.05.2021 Patentblatt 2021/20**

(21) Anmeldenummer: **18713786.4**

(22) Anmeldetag: **28.02.2018**

(51) Int Cl.:
*A61K 9/70* (2006.01)  *A61K 31/122* (2006.01)
*A61P 25/00* (2006.01)

(86) Internationale Anmeldenummer:
**PCT/DE2018/100177**

(87) Internationale Veröffentlichungsnummer:
**WO 2018/157888 (07.09.2018 Gazette 2018/36)**

(54) **TRANSMUKOSALES VERABREICHUNGSSYSTEM FÜR IDEBENON**

TRANSMUCOSAL DELIVERY SYSTEM FOR IDEBENONE

SYSTÈME D'ADMINISTRATION PAR VOIE TRANS-MUQUEUSE D'IDÉBÉNONE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.03.2017 DE 102017104277**

(43) Veröffentlichungstag der Anmeldung:
**08.01.2020 Patentblatt 2020/02**

(73) Patentinhaber:
• **LTS Lohmann Therapie-Systeme AG**
  **56626 Andernach (DE)**
• **Santhera Pharmaceuticals (Schweiz) AG**
  **4133 Pratteln (CH)**

(72) Erfinder:
• **LINN, Michael**
  **55596 Waldböckelheim (DE)**
• **MÜLLER, Markus**
  **53840 Troisdorf (DE)**
• **BAUER, Marius**
  **56626 Andernach (DE)**

(74) Vertreter: **Held, Stephan**
**Meissner Bolte Patentanwälte**
**Rechtsanwälte Partnerschaft mbB**
**Widenmayerstraße 47**
**80538 München (DE)**

(56) Entgegenhaltungen:
EP-A1- 1 891 946     EP-A1- 2 620 141
WO-A1-2013/107810    US-A1- 2014 142 076

EP 3 589 270 B1

**Beschreibung**

Die vorliegende Erfindung betrifft ein transmukosales Verabreichungssystem für Idebenon, ein Verfahren zu dessen Herstellung und die Verwendung eines solchen Systems als Arzneimittel.

Idebenon wird nach der oralen Verabreichung gut im Gastrointestinaltrakt absorbiert und sehr schnell metabolisiert. Es konnte gezeigt werden, dass mehr als 98% Idebenon im "First-Pass-Metabolismus" metabolisiert werden. Die Metabolisierung von Idebenon in der Leber führt zu einer Oxidation der Seitenkette von Idebenon, zur Reduktion des Chinon-Rings, zur Sulfat- und Glukuronidkonjugation und zur anschließenden renale Ausscheidung. Der hohe Leberstoffwechsel reduziert dabei die potentiell hohen Plasmaspiegel des pharmakologisch aktiven Idebenons. Aufgrund dieses starken "First-Pass-Metabolismus" erfordert die orale Verabreichung von Idebenon hohe Dosen der Verbindung, um pharmakologisch wirksame Plasmaspiegel im Körper zu erreichen. Diese hohen Dosen können zu unerwünschten Nebenwirkungen wie Durchfall führen.

Diese Probleme können durch die Verabreichung von Idebenon mittels eines transmukosalen Verabreichungssystems überwunden werden. Transmukosale Verabreichungssysteme sind dünne wirkstoffhaltige Filme auf Polymerbasis, die wenn sie auf eine Schleimhaut, insbesondere die Mundschleimhaut, aufgebracht werden, den Wirkstoff direkt in diese abgeben. Diese Darreichungssysteme haben den Vorteil, dass der Wirkstoff größtenteils durch die Schleimhaut resorbiert wird und damit der "First-Pass Metabolismus", der bei der konventionellen Darreichungsform eines Wirkstoffs in Tablettenform beachtet werden muss, vermieden wird.

Die Verabreichung von Idebenon in Form eines oralen Films ist bekannt.

So offenbart die WO 2013/110442 A1 die Verabreichung von Idebenon als oralen Film auf Basis einer festen Lösung von Idebenon in einem wasserlöslichen Polymer.

Die WO 2013/107810 A1 offenbart ein transmukosales Verabreichungssystem mit einem Trägermaterial auf Basis eines wasserlöslichen Polymers oder eines Cellulosederivats.

Die WO 2008/0197691 A1 beschreibt eine transmukosale Darreichungsform für Idebenon auf Basis einer sich schnell auflösenden Tablette.

Die EP 2 620 141 A1 offenbart ein Dünnschicht-Arzneimittelabgabesystem zur transmukosalen Verabreichung von 1,4-Benzochinonen.

Die EP 1 891 946 A1 offenbart die Verwendung von Idebenon zur Herstellung eines Medikaments zur transmukosalen Verabreichung.

Die US 2014/142076 A1 offenbart Dosierungsformen zur Abgabe von Arzneimitteln über die Mundschleimhaut mit verbesserter transmukosaler Permeabilität.

Die aus dem Stand der Technik bekannten Darreichungsformen für Idebenon auf Basis eines oralen Films aus wasserlöslichen Polymeren haben den Nachteil, dass Idebenon in einer festen Lösung oder einer Suspension in einem wasserlöslichen Polymer vorliegt. Als stark hydrophobe Verbindung liegt Idebenon in festen Lösungen chemisch und/oder physikalisch instabil vor, was eine Rekristallisation von Idebenon zur Folge haben kann. Liegt Idebenon in einer Suspension vor, so kann das den Nachteil einer reduzierten transmukosalen Resorbierbarkeit haben. Wässrige Suspensionen von Idebenon sind aufgrund des niedrigen Schmelzpunktes von Idebenon außerdem schwer herzustellen, da diese nur bei sehr niedrigen Temperaturen getrocknet werden können ohne die physikalischen Eigenschaften des Wirkstoffs (z.B. Partikelgröße) zu verändern, was in Abhängigkeit von der Schichtdicke in sehr langen und nicht umsetzbaren und wirtschaftlich unvorteilhaften Trocknungszeiten resultiert.

Die Aufgabe der vorliegenden Erfindung besteht darin, vorstehend genannte Nachteile des Standes der Technik zu beheben. Insbesondere besteht die Aufgabe der vorliegenden Erfindung darin, ein transmukosales Verabreichungssystem für Idebenon bereitzustellen, wobei Idebenon gelöst sowie chemisch und physikalisch stabil vorliegt, was sich auch positiv auf die transmukosale Resorbierbarkeit auswirken kann. Zudem soll das transmukosale Verabreichungssystem einfach und kostengünstig herzustellen sein.

Obige Aufgabe wird durch ein transmukosales Verabreichungssystem für Idebenon nach Anspruch 1 gelöst.

Das erfindungsgemäße transmukosales Verabreichungssystem für Idebenon liegt vorzugsweise in Form einer flüssig-in-fest Dispersion vor.

Der Wirkstoff wird vorzugsweise in der inneren hydrophoben Phase, die Idebenon und mindestens eine hydrophobe Substanz enthält, gelöst, welche vorzugsweise in Tröpfchenform in einer äußeren festen Phase auf Basis eines wasserlöslichen Polymers dispergiert ist. Zur Stabilisation der Emulsion enthält das transmukosale Verabreichungssystem mindestens einen Emulgator, der die beiden Phasen in Bezug auf Phasentrennung stabilisiert.

Ein solches System hat den Vorteil, dass Idebenon vorzugsweise in der hydrophoben Substanz vorzugsweise in einer Konzentration unterhalb seiner Sättigungskonzentration gelöst werden kann, was ein Auskristallisieren des Wirkstoffs verhindert. Obwohl die Herstellung eines solchen Systems vorzugsweise wasserbasiert erfolgt, kann im Gegensatz zu den Systemen, in denen Idebenon in Form einer festen Lösung oder Suspension vorliegt, eine höhere Trocknungstemperatur gewählt werden, da der Wirkstoff in Lösung und nicht in fester, schmelzfähiger Form vorliegt. Damit werden akzeptable Trocknungszeiten erreicht, die eine kommerzielle, wirtschaftlich akzeptable Fertigung eines solchen Systems ermöglichen.

Unter Idebenon versteht man 2-(10-hydroxydecyl)-5,6-dimethoxy-3-methyl-cyclohexa-2,5-dien-1,4-dion, das beispielsweise unter dem Handelsnamen Raxone® von Santhera Pharmaceuticals vertrieben wird.

In einer bevorzugten Ausführungsform des erfindungsgemäßen transmukosalen Verabreichungssystems liegt Idebenon im Wesentlichen in der inneren hydrophoben Phase des Systems vor. Unter im Wesentlichen wird verstanden, dass Idebenon zu mehr als 80 Gew.-%, bevorzugt mehr als 85 Gew.-% und besonders bevorzugt zu mehr als 90 Gew.-%, bezogen auf die Gesamtmenge an Idebenon in dem System, in der inneren hydrophoben Phase des Systems vorliegt.

Dies hat den Vorteil, dass Idebenon somit im Wesentlichen gelöst und somit chemisch und/oder physikalisch stabil vorliegt. Liegt Idebenon zu weniger als 80 Gew.-% in der inneren hydrophoben Phase vor, so hat das den Nachteil, dass zu viel Idebenon in Form einer festen Lösung oder Suspension in der äußeren hydrophilen Phase vorliegt, was die transmukosale Resorbierbarkeit und die Stabilität des Systems und des Wirkstoffes deutlich beeinträchtigen kann.

In einer bevorzugten Ausführungsform des erfindungsgemäßen transmukosalen Verabreichungssystems liegt Idebenon im Wesentlichen in nicht-kristalliner Form, vorzugsweise im Wesentlichen in der inneren hydrophoben Phase, gelöst vor.

Unter im Wesentlichen wird verstanden, dass Idebenon zu mehr als 80%, bevorzugt zu mehr als 85% und besonders bevorzugt zu mehr als 90 Gew.-% in nicht-kristalliner Form vorliegt und dass dieses nicht-kristalline Idebenon zu mehr als 80 Gew.-%, bevorzugt zu mehr als 85 Gew.-% und besonders bevorzugt zu mehr als 90 Gew.-%, bezogen auf die Gesamtmenge an Idebenon, in der inneren hydrophoben Phase des Systems vorliegt.

Dies hat den Vorteil, dass Idebenon somit im Wesentlichen in nicht-kristalliner Form gelöst vorliegt, was eine bessere transmukosale Resorbierbarkeit zur Folge haben kann.

Liegt Idebenon zu weniger als 80 Gew.-% in nicht-kristalliner Form vor, so hat das den Nachteil, dass die transmukosale Resorbierbarkeit und die Stabilität des Systems und des Wirkstoffes deutlich beeinträchtigt werden.

In einer weiteren bevorzugten Ausführungsform des erfindungsgemäßen transmukosalen Verabreichungssystems liegt Idebenon in einer pharmazeutisch wirksamen Konzentration unterhalb seiner Sättigungskonzentration, vorzugsweise im Wesentlichen in der inneren hydrophoben Phase, vor, vorzugsweise in einer Konzentration, die 90%, bevorzugt 95% der Sättigungskonzentration von Idebenon in der inneren hydrophoben Phase, entspricht. Der Ausdruck "im Wesentlichen" wird hier wie vorstehend definiert verstanden.

Die Sättigungskonzentration von Idebenon in der inneren hydrophoben Phase ist von der Zusammensetzung der inneren hydrophoben Phase abhängig, beträgt in einer besonderen Ausführungsform jedoch von etwa 80 g/L bis etwa 110 g/L bei 4°C, vorzugsweise von etwa 90 g/L bis etwa 105 g/L bei 4°C und von etwa 120 g/L bis etwa 155 g/L bei Raumtemperatur, vorzugsweise von etwa 130 g/L bis etwa 150 g/L bei Raumtemperatur. Die Sättigungskonzentration wird dabei wie folgt bestimmt:

Idebenon wird im Überschuss zur inneren hydrophoben Phase gegeben, so dass auch nach längerem Rühren noch ein fester Bodensatz an Wirkstoff vorhanden ist. Anschließend wird der Bodensatz durch Zentrifugation entfernt. Der erhaltene Überstand enthält Idebenon in seiner Sättigungskonzentration. Diese wird analytisch beispielsweise mittels HPLC bestimmt.

Liegt Idebenon in einer Konzentration unterhalb seiner Sättigungskonzentration vor, so hat das den Vorteil, dass eine Rekristallisation von Idebenon weitgehend verhindert werden kann, was eine deutlich bessere transmukosale Resorbierbarkeit sowie eine verbesserte Stabilität des Wirkstoffes und des Systems zur Folge haben kann.

Die äußere hydrophile Phase des erfindungsgemäßen transmukosalen Verabreichungssystems umfasst mindestens ein hydrophiles Polymer. Ein hydrophiles Polymer ist ein Polymer, dass polare und/oder geladene Gruppen enthält, die das Polymer wasserlöslich machen.

Erfindungsgemäß umfasst das mindestens eine hydrophile Polymer Polyvinylalkohol, Polyvinylpyrrolidon, ein Cellulosederivat und/oder Co-polymere davon.

Diese hydrophilen Polymere haben den Vorteil, dass sie getrocknet einen dünnen stabilen Film bilden, der sich bei Applikation auf der Schleimhaut in einem pharmazeutisch akzeptablen Zeitraum auflöst und damit den Wirkstoff freigibt. Dies hat den Vorteil einer relativ schnellen Verfügbarkeit des Wirkstoffs sowie einer rückstandlosen Darreichung des Wirkstoffes.

Die innere hydrophobe Phase des erfindungsgemäßen transmukosalen Verabreichungssystems umfasst mindestens eine hydrophobe Substanz.

Erfindungsgemäß weist die mindestens eine hydrophobe Substanz in der inneren hydrophoben Phase einen logP-Wert von größer als 1, bevorzugt von größer als 1,5, besonders bevorzugt von größer als 2 auf.

Der n-Octanol-Wasser-Verteilungskoeffizient $K_{ow}$ (auch Schreibweisen wie Octanol/Wasser-Verteilungskoeffizient sind gebräuchlich und korrekt) ist ein dem Fachmann bekannter dimensionsloser Verteilungskoeffizient, der das Verhältnis der Konzentrationen einer Chemikalie in einem Zweiphasensystem aus n-Octanol und Wasser angibt und damit ein Maß für die Hydrophobizität bzw. Hydrophilität eines Stoffes ist. Der logP-Wert ist der dekadische Logarithmus des n-Octanol-Wasser-Verteilungskoeffizienten $K_{ow}$. Dabei gilt:

$$K_{ow} = P = \frac{c_o^{Si}}{c_w^{Si}} \qquad \text{und} \qquad \log P = \log \frac{c_o^{Si}}{c_w^{Si}} = \log c_o^{Si} - c_w^{Si}$$

mit $c_o^{Si}$ = Konzentration einer Chemikalie in der octanolreichen Phase und
$c_w^{Si}$ = Konzentration einer Chemikalie in der wasserreichen Phase.

$K_{ow}$ ist größer als eins, wenn eine Substanz besser in fettähnlichen Lösungsmitteln wie n-Octanol löslich ist, kleiner als eins wenn sie besser in Wasser löslich ist. Entsprechend ist log P positiv für lipophile und negativ für hydrophile Substanzen. Da es sich bei Idebenon um eine sehr hydrophobe Substanz handelt, löst sich Idebenon bevorzugt in einer hydrophoben Substanz mit einen logP-Wert von größer als 1, bevorzugt von größer als 1,5, besonders bevorzugt von größer als 2. In Substanzen mit einem negativen logP-Wert liegt Idebenon lediglich in Form einer Suspension oder in kristalliner Form vor, was wie oben ausgeführt nicht bevorzugt ist.

Erfindungsgemäß umfasst die mindestens eine hydrophobe Substanz in der inneren hydrophoben Phase Tributylcitrat, Triethylcitrat, Eucalyptol, 1,2-Propandiol, Methylsalicylat, Linolsäure, Ölsäure und/oder Mischungen davon.

Das erfindungsgemäße transmukosale Verabreichungssystem umfasst mindestens einen Emulgator. Emulgator ist eine Bezeichnung für Hilfsmittel zur Herstellung und zur Stabilisierung von Emulsionen, die im engeren Sinne auch als grenzflächenaktive Stoffe bzw. Tenside bezeichnet werden können und in der Regel als ölige bis wachsartige, aber auch pulverförmige Stoffe vorliegen. Zur Stabilisierung von Emulsionen über einen längeren Zeitraum werden Hilfsmittel benötigt, die die Entmischung der beiden Phasen Öl und Wasser zum thermodynamisch-stabilen Endzustand unterbinden bzw. so lange verzögern, bis die Emulsion ihre Bestimmung erfüllt hat. Dies kann durch Stabilisatoren und/oder Emulgatoren erreicht werden.

Beispiele für Emulgatoren sind Seifen, Metallseifen, organische Seifen, wie Ethanolaminoleate- oder stearate, sulfurierte Verbindungen, wie Natriumdodecylsulfat, quartäre Ammoniumverbindungen, Fettalkohole, wie Lauryl-, Cetyl-, Stearyl- oder Palmitylalkohol, partielle Fettsäureester mehrwertiger Alkohole mit gesättigten Fettsäuren, wie Glycerolmonostearat, Pentaerythritolmonostearat, Ethylenglycolmonostearat, Propylenglycolmonostearat, partielle Fettsäureester mehrwertiger Alkohole mit ungesättigten Fettsäuren, wie Glycerolmonooleat, Pentaerythritolmonooleat, ferner Polyoxyethylenester von Fettsäuren, wie Polyoxyethylenstearat, Polymerisationsprodukte aus Ethylenoxid und Propylenoxid mit Fettalkoholen, wie Fettalkoholpolyglycolether oder Fettsäuren, wie Fettsäureethoxylate, Polysorbate, Sorbitanester, Mono-oder Diglyceride und/oder Polyoxyethylen-Fettsäureether.

Emulgatoren können über den HLB-Wert charakterisiert werden (HLB = hydrophilic-lipophilic balance = hydrolipophiles Verhältnis). Der HLB-Wert ist ein Maß für die Wasser- bzw. Öllöslichkeit von vorwiegend nichtionischen Tensiden und die Stabilität von Emulsionen.

Der HLB-Wert für nichtionische Tenside kann folgendermaßen berechnet werden

$$HLB = 20 \; x \; \left(1 - \frac{M_l}{M}\right),$$

wobei $M_l$ die Molmasse des lipophilen Anteils eines Moleküls ist und M die Molmasse des gesamten Moleküls. Der Faktor 20 ist ein frei gewählter Skalierungsfaktor. Es ergibt sich damit eine Skala von 0 bis 20. Ein HLB-Wert von 1 spricht für eine lipophile Verbindung, eine chemische Verbindung mit einem HLB-Wert von 20 hat einen hohen hydrophilen Anteil. Ein Wert zwischen 3 und 8 wird Wasser-in-ÖI-Emulgatoren und ein Wert zwischen 8 und 18 Öl-in-Wasser-Emulgatoren zugeordnet.

In einer bevorzugten Ausführungsform ist das transmukosalen Verabreichungssystem dadurch gekennzeichnet, dass der mindestens eine Emulgator einen HLB-Wert von 3 bis 15 aufweist. Emulgatoren mit einem HLB-Wert in diesem Bereich sind besonders gut dazu geeignet, die innere hydrophobe Phase enthaltend Idebenon, in der äußeren hydrophilen Phase, enthaltend mindestens ein hydrophiles Polymer, zu stabilisieren. Emulgatoren mit HLB-Werten von mehr als 15 oder weniger als 3 haben den Nachteil, dass sie keine stabile Emulsion bilden und somit zu instabilen transmukosalen Verabreichungssystemen für Idebenon führen.

In einer bevorzugten Ausführungsform umfasst der mindestens eine Emulgator Polysorbate, Sorbitanester und/oder Polyoxyethylen-Fettsäureether oder Mischungen davon, wie sie beispielsweise unter den Handelsnamen Polysorbat 80, Span 83 bzw. 85 und Brij S2 bekannt sind, ist jedoch nicht auf diese beschränkt.

Weiterhin ist es bevorzugt, dass das erfindungsgemäße transmukosale Verabreichungssystem zusätzlich mindestens einen Radikalfänger und/oder ein Antioxidans umfasst. Bei dem mindestens einem Radikalfänger und/oder Antioxidans handelt es sich um eine chemische Verbindung, die eine unerwünschte Oxidation anderer Substanzen, speziell des Wirkstoffes, verhindert bzw. vermindert und somit einer Alterung des transmukosalen Verabreichungssystems entgegen wirkt. Speziell zeichnen sich Radikalfänger und/oder Antioxidantien dadurch aus, dass sie den durch Luftsauerstoff bewirkten oxidativen Abbau empfindlicher Moleküle, hier speziell des enthaltenen Wirkstoffes, verhindern.

Besonders bevorzugt ist, dass der mindestens eine Radikalfänger Vitamin E.

Der mindestens eine Radikalfänger und/oder das Antioxidans ist vorzugsweise in einer Menge von etwa 0,05 bis etwa 0,2 Gew.-% und bevorzugt von etwa 0,1 bis etwa 0,2 Gew.-%, bezogen auf das Gesamtgewicht des transmukosalen Verabreichungssystems, in dem transmukosalen Verabreichungssystems enthalten.

Außerdem kann das erfindungsgemäße transmukosale Verabreichungssystem zusätzliche dem Fachmann bekannte Additive enthalten. Diese Additive umfassen, Geschmacksstoffe, Farbstoffe, geschmacksmaskierende Stoffe, Permeationsverstärker, Süßstoffe, Füllstoffe, flüssige, vorzugsweise lipophile Hilfsstoffe und/oder pH-Stabilisatoren.

Weiterhin ist es bevorzugt, dass die innere hydrophobe Phase des erfindungsgemäßen transmukosalen Verabreichungssystems etwa 30 bis etwa 60 Gew.-% und bevorzugt etwa 40 bis etwa 50-Gew. %, bezogen auf das Gesamtgewicht des transmukosalen Verabreichungssystems, ausmacht.

Wird die Menge von etwa 60 Gew.-% überschritten, so hat das den Nachteil, dass keine homogene Emulsion mehr hergestellt werden kann. Wird die Menge von etwa 30 Gew.-% unterschritten, so kann die nötige Menge an Wirkstoff nicht mehr in dem erfindungsgemäßen transmukosalen Verabreichungssystems untergebracht werden.

Erfindungsgemäß macht die äußere hydrophile Phase des erfindungsgemäßen transmukosalen Verabreichungssystems etwa 20 bis etwa 70 Gew.-%, bevorzugt etwa 30 bis etwa 40 Gew.-%, bezogen auf das Gesamtgewicht des transmukosalen Verabreichungssystems, aus.

Wird die Menge von etwa 70 Gew.-% überschritten, so hat das den Nachteil, dass dass keine homogene Emulsion mehr hergestellt werden kann.

Wird die Menge von etwa 20 Gew.-% unterschritten, so hat das den Nachteil, dass die nötige Menge an Wirkstoff nicht mehr in dem erfindungsgemäßen transmukosalen Verabreichungssystems untergebracht werden kann.

Weiterhin ist es bevorzugt, dass der Emulgator etwa 2 bis etwa 7 Gew.-% und bevorzugt etwa 3 bis etwa 6,5 Gew.-%, bezogen auf das Gesamtgewicht des erfindungsgemäßen transmukosalen Verabreichungssystems, ausmacht.

Wird die Menge von etwa 7 Gew.-% überschritten, so hat das den Nachteil, dass sich die Emulsion physikalisch verändert.

Wird die Menge von etwa 2 Gew.-% unterschritten, so hat das den Nachteil, dass die hydrophobe Phase nicht in der hydrophilen Phase stabilisiert werden kann.

Das erfindungsgemäße transmukosale Verabreichungssystem für Idebenon besitzt eine Fläche von etwa 0.5 $cm^2$ bis etwa 10 $cm^2$, vorzugsweise von etwa 2 $cm^2$ bis etwa 8 $cm^2$.

Dabei weist das erfindungsgemäße transmukosale Verabreichungssystem ein Flächengewicht von etwa 50 $g/m^2$ bis etwa 250$g/m^2$, bevorzugt von etwa 75 $g/m^2$ bis etwa 225 $g/m^2$ auf.

Dies entspricht einer Schichtdicke von etwa 20 $\mu m$ bis etwa 500 $\mu m$, bevorzugt von etwa 50 $\mu m$ bis etwa 300 $\mu m$.

Das erfindungsgemäße transmukosale Verabreichungssystems für Idebenon löst sich in der Mundhöhle vorzugsweise in einem Zeitraum von weniger als etwa 30 min auf, bevorzugter in einem Zeitraum von weniger als etwa 15 min und ganz besonders bevorzugt in einem Zeitraum von weniger als etwa 10 min.

Die vorliegende Erfindung betrifft ferner ein Verfahren zur Herstellung des erfindungsgemäßen transmukosalen Verabreichungssystems für Idebenon. Das Verfahren umfasst die Schritte

a1) Herstellen einer wässrigen Lösung, umfassend das mindestens eine hydrophile Polymer, wobei das mindestens eine hydrophile Polymer Polyvinylalkohol, Polyvinylpyrrolidon, ein Cellulosederivat und/oder Co-polymere davon umfasst;

a2) Herstellen einer zweiten Lösung, umfassend Idebenon und die mindestens eine hydrophoben Substanz, wobei die mindestens eine hydrophobe Substanz in der inneren hydrophoben Phase Methylsalicylat, Tributylcitrat, Triethylcitrat, Eucalyptol, 1,2-Propandiol, Linolsäure, Ölsäure und/oder Mischungen davon umfasst und wobei mindestens eine der beiden Lösungen der Schritte a1) bzw. a2) zusätzlich den mindestens einen Emulgator umfasst;

b) Mischen der beiden Lösungen aus den Schritten a1) und a2), um eine Emulsion zu erhalten, wobei die äußere hydrophile Phase 20 bis 70 Gew.-%, bezogen auf das Gesamtgewicht des transmukosalen Verabreichungssystems für Idebenon, ausmacht; und

c) Ausstreichen und Trocknen der gemäß Schritt b) erhaltenen Emulsion, so dass die getrocknete Emulsion ein Flächengewicht von etwa 50 bis etwa 250 $g/m^2$ aufweist.

Die vorliegende Erfindung betrifft ferner ein transmukosales Verabreichungssystems für Idebenon erhältlich nach dem oben beschriebenen Verfahren.

Schließlich betrifft die vorliegende Erfindung die vorstehend näher beschriebenen transmukosalen Verabreichungssysteme für Idebenon und die transmukosalen Verabreichungssysteme für Idebenon, erhältlich nach dem vorstehend beschriebenen Verfahren als Arzneimittel.

Ferner betrifft die vorliegende Erfindung ein transmukosales Verabreichungssystems für Idebenon in der Behandlung von Friedreichscher-Ataxie, Duchenne-Muskeldystrophie, primär progredienter Multiple Sklerose, lebersche heriditäre Optikusneuropathie.

Die Erfindung wird nachfolgend anhand von nicht beschränkenden Beispielen erläutert.

Beispiel 1:

| Probe | PVA 4-88 [%] | Emulgator [%] | HLB-Wert des Emulgators | Innere hydrophobe Phase | | | | Sonstiges [%] | Evaluation |
|---|---|---|---|---|---|---|---|---|---|
| | | | | MS [%] LogP = 2,24 | DMI [%] LogP = -0,6 | TBC [%] LogP = 4,7 | EUC [%] LogP = 2,8 | | |
| 38 | 39,90 | --- | | --- | 25,00 | 25,00 | --- | 0,10 Vitamin E | Keine Emulsion |
| 45 | 38,80 | 4,74 PS80 | 15,0 | 47,46 | --- | --- | --- | 0,11 Vitamin E | Gut |
| 46 | 39,77 | --- | | 5,05 | 25,08 | --- | --- | 0,11 Vitamin E | PhysikalischeVeränderung |
| 47 | 38,32 | 4,19 PS80 | 15,0 | 23,86 | --- | --- | 23,93 | 0,14 Vitamin E | Gut |
| 48 | 37,92 | 5,31 PS80 | 15,0 | 23,86 | --- | 23,58 | --- | 0,12 Vitamin E | Gut |
| 49 | 39,95 | --- | | --- | 49,93 | --- | --- | 0,13 Vitamin E | In homogen |
| 50 | 39,59 | --- | | --- | 24,73 | --- | 24,65 | 0,12 Vitamin E | PhysikalischeVeränderung |
| 51 | 39,89 | --- | | --- | 27,97 | 25,02 | --- | 0,11 Vitamin E | PhysikalischeVeränderung |
| 52 | 39,55 | 1,97 PS80 | 15,0 | --- | --- | --- | 48,56 | 0,15 Vitamin E | Leicht Inhomogen |
| 53 | 39,42 | 5,45 PS80 | 15,0 | --- | --- | 22,92 | 22,94 | 0,10 Vitamin E | Gut |
| 54 | 37,34 | 6,79 PS80 | 15,0 | --- | --- | 46,43 | --- | 0,11 Vitamin E | In homogen |
| 55 | 37,50 | 5,00 PS80 | 15,0 | 47,40 | --- | --- | --- | 0,10 Vitamin E | Gut |
| 56 | 36,40 | 8,00 PS80 | 15,0 | --- | --- | 45,50 | --- | 0,10 Vitamin E | PhysikalischeVeränderung |
| 57 | 39,50 | 1,00 PS80 | 15,0 | --- | 49,40 | --- | --- | 0,10 Vitamin E | In homogen |
| 58 | 36,90 | 5,00 PS80 | 15,0 | 24,00 | --- | --- | 24,00 | 0,10 Vitamin E | Gut |
| 59 | 35,90 | 6,00 PS80 | 15,0 | 24,00 | --- | 24,00 | --- | 0,10 Vitamin E | Gut |
| 60 | 39,60 | 6,00 PS80 | 15,0 | 14,00 | --- | 19,00 | 14,00 | 0,10 Vitamin E | Gut |
| 61 | 35,90 | 6,00 PS80 | 15,0 | 14,00 | 5,00 | 15,00 | 14,00 | 0,10 Vitamin E | Gut |
| 64 | 32,10 | 6,00 Atmos 300 6,00 PS80 | 2,5 15,0 | 13,00 | --- | 10,00 | 13,00 | 0,10 Vitamin E 0,10 Vit.E.TPGS 4,50 Sucralose 0,20 Patentblau | In homogen |
| 75 | 36,90 | 6,00 PS80 | 15,0 | 10,00 | --- | 19,00 | 18,00 | 0,10 Vitamin E | Gut |
| 76 | 36,90 | 6,00 BRij S2 | 4,90 | 10,00 | --- | 19,00 | 18,00 | 0,10 Vitamin E | Gut |

(fortgesetzt)

| Probe | PVA 4-88 [%] | Emulgator [%] | HLB-Wert des Emulgators | Innere hydrophobe Phase | | | | Sonstiges [%] | Evaluation |
|---|---|---|---|---|---|---|---|---|---|
| | | | | MS [%] $LogP = 2,24$ | DMI [%] $LogP = -0,6$ | TBC [%] $LogP = 4,7$ | EUC [%] $LogP = 2,8$ | | |
| 77 | 36,90 | 4,00 PS80 2,00 Brij S2 | 15,0 4,9 | 10,00 | --- | 19,00 | 18,00 | 0,10 Vitamin E | Gut |
| 78 | 37,40 | 3,50 PS80 1,50 Brij S2 | 15,0 4,90 | 9,00 | --- | 17,00 | 16,00 | 0,10 Vitamin E 1,00 Lemon Lime 3,00 Peppermint 1,50 Sucralose | Gut |
| 79 | 37,00 | 3,50 PS80 1,50 Brij S2 | 15,0 4,90 | 9,00 | --- | 15,00 | 14,40 | 0,10 Vitamin E 5,00 Spearmint 3,00 Mask Flavour 1.50 Sucralose | Gut |

PVA 4-88: Polyvinylalkohol (äußere hydrophile Phase)
PS80: Polysorbat 80
MS: Methylsalicylat
TBC: Tributylcitrat
EUC: Eucalyptol
DMI: Dimethylisosorbid
Atmos 300: Mono und Diglyceride

Die erfindungsgemäßen Beispiele 45, 47, 48, 53, 58 bis 61 und 75 bis 78 bilden alle eine physikalisch und chemisch stabile Emulsion. Vergleichsbeispiel 38, das keinen Emulgator und keine hydrophobe Substanz enthält, bildet keine Emulsion. Die Vergleichsbeispiele 49 bis 51, die keinen Emulgator enthalten, sind entweder inhomogen oder zeigen physikalische Veränderungen. Vergleichsbeispiel 52 zeigt eine leichte Inhomogenität, da es nicht genug Emulgator enthält. Die Vergleichsbeispiele 54, 56 und 57 sind inhomogen, da sie keine hydrophobe Substanz enthalten. Vergleichsbeispiel 64 enthält den nicht erfindungsgemäßen Emulgator ATMOS 300, dessen Einsatz zu einer inhomogenen Emulsion führt.

Beispiel 2:

Die Sättigungskonzentration von Idebenon für eine beispielhafte erfindungsgemäße Zusammensetzung wurde wie folgt bestimmt. Idebenon wird im Überschuss zur inneren hydrophoben Phase gegeben, so dass auch nach längerem Rühren noch ein fester Bodensatz an Wirkstoff vorhanden ist. Anschließend wird der Bodensatz durch Zentrifugation entfernt. Der erhaltene Überstand enthält Idebenon in seiner Sättigungskonzentration. Diese wird analytisch beispielsweise mittels HPLC bestimmt.

| Menge [Gew.-%] | Ingredienz |
|---|---|
| 0,17 | Vitamin E |
| 6,74 | Polysorbat 80 |
| 19,29 | Methylsalicylat |
| 34,46 | Eucalyptol |
| 36,44 | Tributylcitrat |
| 2,89 | BrijS2 |

In obiger Zusammensetzung weist Idebenon eine Sättigungskonzentration von 145 g/L bei Raumtemperatur und von 103 g/L bei 4°C auf.

## Patentansprüche

1. Transmukosales Verabreichungssystem für Idebenon, umfassend eine äußere hydrophile Phase, die mindestens ein hydrophiles Polymer enthält, wobei das mindestens eine hydrophile Polymer Polyvinylalkohol, Polyvinylpyrrolidon, ein Cellulosederivat und/oder Co-polymere davon umfasst und wobei die äußere hydrophile Phase 20 bis 70 Gew.-%, bezogen auf das Gesamtgewicht des transmukosalen Verabreichungssystems für Idebenon, ausmacht und eine innere hydrophobe Phase, die Idebenon und mindestens eine hydrophobe Substanz enthält, wobei die mindestens eine hydrophobe Substanz in der inneren hydrophoben Phase Methylsalicylat, Tributylcitrat, Triethylcitrat, Eucalyptol, 1,2-Propandiol, Linolsäure, Ölsäure und/oder Mischungen davon umfasst und wobei die innere hydrophobe Phase in Form von Tröpfchen in der äußeren Phase emulgiert ist, **dadurch gekennzeichnet, dass** die innere hydrophobe Phase durch mindestens einen Emulgator in der äußeren Phase stabilisiert ist.

2. Transmukosales Verabreichungssystem für Idebenon gemäß Anspruch 1, **dadurch gekennzeichnet, dass** Idebenon im Wesentlichen in der inneren hydrophoben Phase vorliegt.

3. Transmukosales Verabreichungssystem für Idebenon gemäß Anspruch 1 oder 2, **dadurch gekennzeichnet, dass** Idebenon in nicht-kristalliner Form, vorzugsweise im Wesentlichen in der inneren hydrophoben Phase, gelöst vorliegt.

4. Transmukosales Verabreichungssystem für Idebenon gemäß irgendeinem der vorhergehenden Ansprüche, **dadurch gekennzeichnet, dass** Idebenon in einer pharmazeutisch wirksamen Konzentration unterhalb seiner Sättigungskonzentration, vorzugsweise im Wesentlichen in der inneren hydrophoben Phase, vorliegt, vorzugsweise in einer Konzentration, die 90% der Sättigungskonzentration von Idebenon in der mindestens einen hydrophoben Substanz entspricht.

5. Transmukosales Verabreichungssystem für Idebenon gemäß irgendeinem der vorhergehenden Ansprüche, **dadurch gekennzeichnet, dass** der mindestens eine Emulgator einen HLB-Wert von 3 bis 15 aufweist.

6. Transmukosales Verabreichungssystem für Idebenon gemäß irgendeinem der vorhergehenden Ansprüche, **dadurch gekennzeichnet, dass** der mindestens eine Emulgator Polysorbat, Sorbitanester, Polyoxyethylen-Fettsäureether und/oder Mischungen davon umfasst.

7. Transmukosales Verabreichungssystem für Idebenon gemäß irgendeinem der vorhergehenden Ansprüche, **dadurch gekennzeichnet, dass** das transmukosale Verabreichungssystem für Idebenon zusätzlich mindestens einen Radikalfänger umfasst.

8. Transmukosales Verabreichungssystem für Idebenon gemäß Anspruch 10, **dadurch gekennzeichnet, dass** der mindestens eine Radikalfänger Vitamin E umfasst.

9. Transmukosales Verabreichungssystem für Idebenon gemäß irgendeinem der vorhergehenden Ansprüche, **dadurch gekennzeichnet, dass** die innere Phase 30 bis 60 Gew.-%, bezogen auf das Gesamtgewicht des transmukosalen Verabreichungssystems für Idebenon, ausmacht.

10. Transmukosales Verabreichungssystem für Idebenon gemäß irgendeinem der vorhergehenden Ansprüche, **dadurch gekennzeichnet, dass** der Emulgator 2 bis 7 Gew.-%, bezogen auf das Gesamtgewicht des transmukosalen Verabreichungssystems für Idebenon, ausmacht.

11. Verfahren zur Herstellung eines transmukosalen Verabreichungssystems für Idebenon gemäß irgendeinem der Ansprüche 1 bis 10, umfassend die Schritte:

a1) Herstellen einer wässrigen Lösung, umfassend das mindestens eine hydrophile Polymer, wobei das mindestens eine hydrophile Polymer Polyvinylalkohol, Polyvinylpyrrolidon, ein Cellulosederivat und/oder Co-polymere davon umfasst;
a2) Herstellen einer Lösung, umfassend Idebenon und die mindestens eine hydrophoben Substanz, wobei die mindestens eine hydrophobe Substanz in der inneren hydrophoben Phase Methylsalicylat, Tributylcitrat, Triethylcitrat, Eucalyptol, 1,2-Propandiol, Linolsäure, Ölsäure und/oder Mischungen davon umfasst; und wobei mindestens eine der beiden Lösungen der Schritte a1) bzw. a2) zusätzlich den mindestens einen Emulgator umfasst;
b) Mischen der beiden Lösungen aus den Schritten a1) und a2), um eine Emulsion zu erhalten, wobei die äußere hydrophile Phase 20 bis 70 Gew.-%, bezogen auf das Gesamtgewicht des transmukosalen Verabreichungssystems für Idebenon, ausmacht; und
c) Ausstreichen und Trocknen der gemäß Schritt b) erhaltenen Emulsion, so dass die getrocknete Emulsion ein Flächengewicht von etwa 50 bis etwa 250 g/m$^2$ aufweist.

12. Transmukosales Verabreichungssystem für Idebenon, erhältlich nach dem Verfahren gemäß Anspruch 11.

13. Verwendung des transmukosalen Verabreichungssystem für Idebenon gemäß den Ansprüchen 1 bis 10 und 12 als Arzneimittel, insbesondere in der Behandlung von Friedreichscher-Ataxie, Duchenne-Muskeldystrophie, primär progredienter Multiple Sklerose, lebersche heriditäre Optikusneuropathie.

**Claims**

1. A transmucosal delivery system for idebenone, comprising an outer, hydrophilic, phase which contains at least one hydrophilic polymer, wherein the at least one hydrophilic polymer comprises polyvinyl alcohol, polyvinylpyrrolidone, a cellulose derivative and/or copolymers thereof and wherein and the outer, hydrophilic, phase makes up 20 to 70 wt.%, relative to the total weight of the transmucosal delivery system for idebenone, and an inner hydrophobic phase which contains idebenone and at least one hydrophobic substance, wherein the at least one hydrophobic substance in the inner, hydrophobic, phase comprises methyl salicylate, tributyl citrate, triethyl citrate, eucalyptol, 1,2-propanediol, methyl salicylate, linoleic acid, oleic acid and/or mixtures thereof, and wherein the inner, hydrophobic, phase is emulsified in the form of droplets in the outer phase, **characterised in that** the inner, hydrophobic, phase is stabilised by at least one emulsifier in the outer phase.

2. A transmucosal delivery system for idebenone according to Claim 1, **characterised in that** idebenone is present substantially in the inner, hydrophobic, phase.

3. A transmucosal delivery system for idebenone according to Claim 1 or Claim 2, **characterised in that** idebenone is present dissolved in non-crystalline form, preferably substantially in the inner, hydrophobic, phase.

4. A transmucosal delivery system for idebenone according to any one of the preceding claims, **characterised in that** idebenone is present in a pharmaceutically effective concentration below its saturation concentration, preferably substantially in the inner, hydrophobic, phase, preferably in a concentration which corresponds to 90% of the saturation concentration of idebenone in the at least one hydrophobic substance.

5. A transmucosal delivery system for idebenone according to any one of the preceding claims, **characterised in that** the at least one emulsifier has an HLB value of 3 to 15.

6. A transmucosal delivery system for idebenone according to any one of the preceding claims, **characterised in that** the at least one emulsifier comprises polysorbate, sorbitan esters, polyoxyethylene fatty acid ethers and/or mixtures thereof.

7. A transmucosal delivery system for idebenone according to any one of the preceding claims, **characterised in that** the transmucosal delivery system for idebenone additionally comprises at least one free-radical scavenger.

8. A transmucosal delivery system for idebenone according to Claim 10, **characterised in that** the at least one free-radical scavenger comprises vitamin E.

9. A transmucosal delivery system for idebenone according to any one of the preceding claims, **characterised in that** the inner phase makes up 30 to 60 wt.%, relative to the total weight of the transmucosal delivery system for idebenone.

10. A transmucosal delivery system for idebenone according to any one of the preceding claims, **characterised in that** the emulsifier makes up 2 to 7 wt.%, relative to the total weight of the transmucosal delivery system for idebenone.

11. A method for producing a transmucosal delivery system for idebenone according to any one of Claims 1 to 10, comprising the steps:

a1) producing an aqueous solution, comprising the at least one hydrophilic polymer wherein the at least one hydrophilic polymer comprises polyvinyl alcohol, polyvinylpyrrolidone, a cellulose derivative and/or copolymers thereof;
a2) producing a solution, comprising idebenone and the at least one hydrophobic substance, wherein the at least one hydrophobic substance in the inner, hydrophobic, phase comprises methyl salicylate, tributyl citrate, triethyl citrate, eucalyptol, 1,2-propanediol, methyl salicylate, linoleic acid, oleic acid and/or mixtures thereof, wherein at least one of the two solutions of steps a1) or a2) additionally comprises the at least one emulsifier;
b) mixing the two solutions from steps a1) and a2) in order to obtain an emulsion, wherein the outer, hydrophobic, phase makes up 20 to 70 wt.%, relative to the total weight of the transmucosal delivery system for idebenone; and
c) spreading out and drying the emulsion obtained in step b) so that the dried emulsion has a weight per unit area of about 50 to about 250 g/m$^2$.

12. A transmucosal delivery system for idebenone, obtainable by the method according to Claim 11.

13. Use of the transmucosal delivery system for idebenone according to claims 1 to 10 and 12 as a medicament, especially in the treatment of Friedreich's ataxia, Duchenne muscular dystrophy, primary progressive multiple sclerosis, Leber's hereditary optic neuropathy.

**Revendications**

1. Système d'administration transmuqueuse pour de l'idébénone, comprenant une phase hydrophile externe, qui contient au moins un polymère hydrophile, dans lequel l'au moins un polymère hydrophile comprend de l'alcool polyvinylique, de la polyvinylpyrrolidone, un dérivé cellulosique et/ou des copolymères de ceux-ci et dans lequel la phase hydrophile externe représente 20 à 70 % en poids, par rapport au poids total du système d'administration transmuqueuse pour de l'idébénone, et une phase hydrophobe interne, qui contient de l'idébénone et au moins une substance hydrophobe, dans lequel l'au moins une substance hydrophobe dans la phase hydrophobe interne comprend du salicylate de méthyle, du citrate de tributyle, du citrate de triéthyle, de l'eucalyptol, du 1,2-propandiol, de l'acide linoléique, de l'acide oléique et/ou des mélanges de ceux-ci et dans lequel la phase hydrophobe interne est émulsifiée sous forme de gouttelettes dans la phase externe, **caractérisé en ce que** la phase hydrophobe interne est stabilisée par au moins un émulsifiant dans la phase externe.

2. Système d'administration transmuqueuse pour de l'idébénone selon la revendication 1, **caractérisé en ce que** de l'idébénone est présente sensiblement dans la phase hydrophobe interne.

3. Système d'administration transmuqueuse pour de l'idébénone selon la revendication 1 ou 2, **caractérisé en ce que** de l'idébénone est présente sous forme non cristalline, de préférence sensiblement dans la phase hydrophobe interne.

4. Système d'administration transmuqueuse pour de l'idébénone selon l'une quelconque des revendications précédentes, **caractérisé en ce que** de l'idébénone est présente dans une concentration à efficacité pharmaceutique au-dessous de sa concentration de saturation, de préférence sensiblement dans la phase hydrophobe interne, de préférence dans une concentration qui correspond à 90 % de la concentration de saturation d'idébénone dans l'au moins une substance hydrophobe.

5. Système d'administration transmuqueuse pour de l'idébénone selon l'une quelconque des revendications précédentes, **caractérisé en ce que** l'au moins un émulsifiant présente une HLB de 3 à 15.

6. Système d'administration transmuqueuse pour de l'idébénone selon l'une quelconque des revendications précédentes, **caractérisé en ce que** l'au moins un émulsifiant comprend du polysorbate, de l'ester de sorbitane, de l'éther d'acide gras de polyoxyéthylène et/ou des mélanges de ceux-ci.

7. Système d'administration transmuqueuse pour de l'idébénone selon l'une quelconque des revendications précédentes, **caractérisé en ce que** le système d'administration transmuqueuse pour de l'idébénone comprend en plus au moins un capteur de radicaux libres.

8. Système d'administration transmuqueuse pour de l'idébénone selon la revendication 10, **caractérisé en ce que** l'au moins un capteur de radicaux libres comprend la vitamine E.

9. Système d'administration transmuqueuse pour de l'idébénone selon l'une quelconque des revendications précédentes, **caractérisé en ce que** la phase interne représente 30 à 60 %, par rapport au poids total du système d'administration transmuqueuse pour de l'idébénone.

10. Système d'administration transmuqueuse pour de l'idébénone selon l'une quelconque des revendications précédentes, **caractérisé en ce que** l'émulsifiant représente 2 à 7 % en poids, par rapport au poids total du système d'administration transmuqueuse pour de l'idébénone.

11. Procédé pour fabriquer un système d'administration transmuqueuse pour de l'idébénone selon l'une quelconque des revendications 1 à 10, comprenant les étapes suivantes :

   a1) fabrication d'une solution aqueuse, comprenant l'au moins un polymère hydrophile, dans lequel l'au moins un polymère hydrophile comprend de l'alcool polyvinylique, de la polyvinylpyrrolidone, un dérivé cellulosique et/ou des copolymères de ceux-ci ;
   a2) fabrication d'une solution, comprenant de l'idébénone et l'au moins une substance hydrophobe, dans lequel l'au moins une substance hydrophobe dans la phase hydrophobe interne comprend du salicylate de méthyle, du citrate de tributyle, du citrate de triéthyle, de l'eucalyptol, du 1,2-propandiol, de l'acide linoléique, de l'acide oléique et/ou des mélanges de ceux-ci ; et

dans lequel au moins une des deux solutions des étapes a1) ou a2) comprend en plus l'au moins un émulsifiant ;

b) mélange des deux solutions provenant des étapes a1) et a2) afin d'obtenir une émulsion, dans lequel la phase hydrophile externe représente 20 à 70 % en poids, par rapport au poids total du système d'administration transmuqueuse pour de l'idébénone ; et

c) étalement et séchage de l'émulsion obtenue selon l'étape b), de sorte que l'émulsion séchée présente une masse surfacique de sensiblement 50 à sensiblement 250 g/m$^2$.

12. Système d'administration transmuqueuse pour de l'idébénone, pouvant être obtenu d'après le procédé selon la revendication 11.

13. Utilisation du système d'administration transmuqueuse pour de l'idébénone selon les revendications 1 à 10 et 12 en tant que médicament, en particulier dans le traitement de l'ataxie de Friedreich, la dystrophie musculaire de Duchenne, la sclérose en plaques progressive primaire, la neuropathie optique héréditaire de Leber.

EP 3 589 270 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013110442 A1
- WO 2013107810 A1
- WO 20080197691 A1

- EP 2620141 A1
- EP 1891946 A1
- US 2014142076 A1